# EUROPEAN PATENT SPECIFICATION

(11) **EP 4 114 364 B1**
(45) Date of publication and mention of the grant of the patent: **30.10.2024**
(21) Application number: 21709376.4
(22) Date of filing: 01.03.2021
(51) Int. Cl.: A61K 9/20

(54) **FORMULATION OF LISINOPRIL AND AMLODIPINE**
FORMULIERUNG VON LISINOPRIL UND AMLODIPIN
FORMULATION DE LISINOPRIL ET D'AMLODIPINE

(30) Priority: 03.03.2020 GB 202003048
(43) Date of publication of application: 11.01.2023
(73) Proprietor: Alkaloid AD Skopje, 1000 Skopje (MK)
(72) Inventor: ALI MEMED, Oja, 1000 Skopje (MK); ANEVSKA-STOJANOVSKA, Natasa, 1000 Skopje (MK); ATANASOVA, Ana, 1000 Skopje (MK); VANOVA, Nadica, 1400 Veles (MK)
(74) Representative: CSY London
(86) International application number: PCT/EP2021/054986
(87) International publication number: WO 2021/175755

(56) References cited:
- EP-B1- 1 765 342
- CN-A- 106 333 948
- PEIKOVA: "Investigations and HPLC assay of model formulations containing amlodipine besylate and lisinopril", vol. 20, no. 1, 1 May 2013 (2013-05-01), pages 11 - 15, XP009527557, ISSN: 0976-044X, Retrieved from the Internet <URL:https://www.globalresearchonline.net/pharmajournal/vol20iss1.aspx>

## Description

The present invention relates to novel pharmaceutical formulations, to methods of preparing them, methods of treatment employing them, and unit dosage formats incorporating them. In specific embodiments, the invention relates to formulations of lisinopril and amlodipine.

### Background of the Invention

Amlodipine, sold under the brand name Norvasc among others, is a medication used to treat high blood pressure and coronary artery disease. Amlodipine may be used if other medications are not sufficient for treating high blood pressure or heart-related chest pain. Amlodipine was patented in 1982 in inter alia U.S. Pat. No. 4,572,909 and approved for medical use in 1990. The IUPAC name of amlodipine is ±2-[(2-aminoethoxy)methyl]-4-(2-chlorophenyl)-1 ,4-dihydro-6-methyl-3,5-pyridinedicarboxylic acid 3-ethyl 5-methyl ester, and it has the chemical structure:

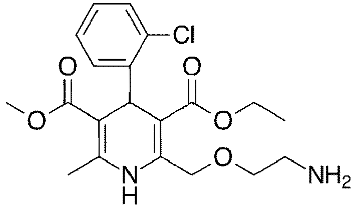

Lisinopril is a medication of the angiotensin-converting enzyme (ACE) inhibitor class used to treat high blood pressure, heart failure, and after heart attacks. This compound, as well as therapeutically acceptable salts thereof, are described in *inter alia* United States patent serial No. 4,374,829 (Merck & Co. Inc.). The IUPAC name of lisinopril is N²-[(1S)-1-carboxy-3-phenylpropyl]-L-lysyl-L-proline. Its structural formula is as follows:

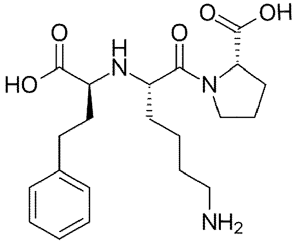

### Prior Art

Combined preparations of lisinopril and amlodipine are known for example from J Hypertens. 1993 Aug;11 (8):839-47. A fixed-dose combination is sold under the brand name "Lisonorm" (among others), and is used to treat high blood pressure.

Formulation of compositions comprising lisinopril is known to be challenging. On long-term storage, particularly with low dosage formulations, lisinopril has a tendency to form diketopiperazine (S,S,S-DKP), a lisinopril degradation product or metabolite, which limits the shelf life for low dose formulations.

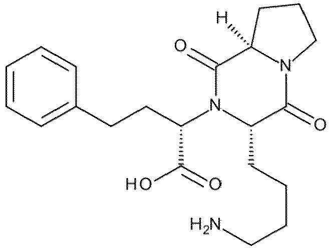

EP1765342B1 relates to a pharmaceutical composition containing 0.3-30 wt % (m/m) of amlopidine besylate and 0.1-75 wt %(m/m) of lisinopril dihydrate as active ingredients in admixture with one or more excipient(s) commonly used in the pharmaceutical industry with the proviso that said excipient is other than a calcium salt of phosphoric acid. This document explains that calcium phosphate should not be used in the case of the formulation of lisinopril dihydrate as pharmaceutically active ingredient due to the large difference in bulk densities. The characteristic bulk density of calcium phosphate is far greater than the characteristic bulk density of lisinopril dihydrate. It is also made clear in this document that the wet granulated mass must be dried *in vacuo* at a temperature below 35-40 °C to avoid degradation of the product.

Int. J. Pharm. Sci. Rev. Res., 20(1) discloses a formulation of lisinopril and amlodipine with calcium phosphate dibasic anhydrous (Table 3, Formulation M2). The amount of calcium phosphate dibasic anhydrous in this formulation is high (approx. 66 %).

US6,462,022B1 discloses pharmaceutical formulations comprising dibasic calcium phosphate dihydrate (DCPD) and lisinopril. The use of large particle size DCPD is said to retard the formation of the lisinopril degradation product DKP. The DCPD is present in an amount of from 30-95 % by weight.

Belarussian patent BY18677C1 discloses an antihypertensive medicine containing amlodipine besilate, lisinopril dihydrate, calcium hydrophosphate, microcrystalline cellulose, hydroxy-propyl methylcellulose, sodium starch glycolate, sodium stearyl fumarate and aerosol. Calcium hydrophosphate is present in an amount of 1.0 wt%.

There remains an unmet need for formulations of lisinopril and amlodipine which do not require heat-controlled drying *in vacuo* as part of the formulation process.

There remains an unmet need for formulations of lisinopril and amlodipine which dissolve more rapidly than known formulations.

There remains a need for a formulation of lisinopril and amlodipine which can be more easily compressed into tablets of required shape and size, at lower compression forces.

There remains a need for a formulation of lisinopril and amlodipine which is more storage stable than those known in the art.

The present invention addresses these and other problems of the prior art.

### Summary of the Invention

The invention is according to the appended claims.

According to a first embodiment, the invention provides a pharmaceutical formulation comprising
a) a first active ingredient which is lisinopril or a salt or hydrate thereof and is unstable;
b) a second active ingredient which is amlodipine or a salt or hydrate thereof;
c) a bulk diluent;
d) optionally, one or more further adjuvants and/or excipients; and
e) dibasic calcium phosphate anhydrous in an amount of from 2 to 5 % w/w.

According to a second embodiment, there is provided a pharmaceutical formulation as hereinbefore described for use in therapy.

According to a third embodiment, there is provided a pharmaceutical formulation as hereinbefore described for the treatment of hypertension. According to a fourth embodiment, the invention relates to a process for the preparation of a pharmaceutical formulation as hereinbefore defined, comprising the steps of
a) combining the first active ingredient, bulk diluent, and optionally, one or more further adjuvants and/or excipients and homogenizing;
b) adding a binder solution;
c) granulating the product of step b);
d) drying at atmospheric pressure;
e) adding the second active ingredient to form a bulk formulation.

### Brief description of the Figures

Figure 1 is a flow chart
Figures 2-4 are graphs comparing the solubility of tablets according to the invention with those of the prior art.

### Detailed Description of the Preferred Embodiments

Surprisingly, it has been found that inclusion of dibasic calcium phosphate anhydrous in amounts of from 2 to 5 % w/w can have a surprising stabilising and thermo protective effect towards formulations comprising lisinopril and amlodipine.

Dibasic calcium phosphate is the calcium phosphate with the formula CaHPO₄. It is also known as dicalcium phosphate, calcium monohydrogen phosphate, and Dicafos. Three forms of dicalcium phosphate are known: dihydrate, CaHPO₄•2H₂O ('DPCD'), the mineral brushite; hemihydrate, CaHPO₄•0.5H₂O and anhydrous CaHPO₄, ('DCPA'), the mineral monetite. Of these, the present invention concerns anhydrous CaHPO₄.

Dibasic calcium phosphate anhydrous is available in various grades, distinguished by particle size. The median particle size of the dibasic calcium phosphate anhydrous is desirably not more than 20 µm and in other embodiments of the invention is not more than 19, 18, 17, 16, 15, 14, 13, 12, 11, 10, 9, 8 or 7 µm. In one embodiment, the median particle size of the dibasic calcium phosphate anhydrous is about 1 to about 10 µm, e.g., about 7 µm. The particle size of dibasic calcium phosphate anhydrous may be varied as desired using any of the conventional techniques such as milling, grinding, attrition, and/or sieving. The median particle size can be measured by using, for example, a laser diffraction/scattering particle size distribution analyzer (e.g., Mastersizer 2000, manufactured by Malvern Instruments) and can be calculated as the midpoint of the particle size distribution, such that half the particles are larger in size than the D50 value and half the particles are smaller in size than the D50 value. In a preferred embodiment, less than 1 % of the dibasic calcium phosphate anhydrous particles have a size of 45 µm or greater when measured using a 325 Mesh (45 µm) sieve. In a more preferred embodiment, less than 0.1 % of the dibasic calcium phosphate anhydrous particles have a size of 45 µm or greater when measured using a 325 Mesh (45 µm) sieve.

The amount of dibasic calcium phosphate present in the pharmaceutical formulation is low compared to instances where it is used as bulk diluent. The amount is from 2 to 5 % based on the weight of the formulation as a whole (w/w). In larger quantities, it can have a deleterious effect on the dissolution characteristics, whilst in smaller quantities, the stabilising effect is not evident. More preferably, the amount is from 2 to 3 % (w/w). Most preferably, the amount is about 2.5 % (w/w).

The formulations of the invention also comprise a bulk diluent or filler. Diluents act as fillers in pharmaceutical tablets to increase weight and improve content uniformity. Natural diluents include starches, hydrolyzed starches, and partially pregelatinized starches. Common diluents include anhydrous lactose, lactose monohydrate, and sugar alcohols (polyols) such as sorbitol, xylitol and mannitol. Preferably, the bulk diluent is a polyol. More preferably, the polyol is selected from the group of isomalt, lactitol, mannitol, sorbitol and xylitol. Most preferably, the bulk diluent is mannitol.

The formulations of the invention may comprise various other pharmaceutical adjuvants and excipients. Pharmaceutically acceptable excipients are well known to those skilled in the art and are disclosed for example, in Staniforth, U.S. Pat. No. 6,936,277, and Lee, U.S. Pat. No. 6,936,628. Excipients such as diluents, binders, glidants, and lubricants are added as processing aids to make the tableting operation more effective. Still other types of excipients enhance or retard the rate of disintegration of the tablet, improve the taste of the tablet (for example, sweetening agents), or impart a colour or flavour to the tablets.

One or more lubricants may be added to a tablet formulation comprising the particulate product of the present invention to prevent the formulation from sticking to the punches during tablet manufacture. Suitable lubricants include, for example, fatty acids, fatty acid salts, and fatty acid esters such as magnesium stearate, calcium stearate, stearic acid, sodium stearyl fumarate, hydrogenated vegetable oil and the like. Lubricants may typically comprise about 0.1 wt % to about 3.0 wt % or about 0.5 wt % to about 2 wt %, most preferably about 1.4 % to about 1.6 %, such as about 1.5 % of the formulation. Magnesium stearate is preferred.

Antiadherents may be utilized to prevent sticking of the tablet formulation to the punch face and die wall. They are used in combination with magnesium stearate when sticking is a problem. Commonly used antiadherents are cornstarch and talc.

One or more binders in addition to the particulate product of the present invention may be added to further modify the cohesive qualities of the powdered material(s). Suitable additional binders include starch, microcrystalline cellulose, and sugars such as sucrose, glucose, dextrose, and lactose. Partially pregelatinized maize starch is preferred.

The amount of binder is preferably from 1 to 20 %, more preferably from 5 to 15 %, more preferably from 8 to 12 % such as about 10 % w/w.

Additionally, one or more disintegrants may also be included in the tablet formulation to ensure that the tablet has an acceptable dissolution rate in an environment of use (such as the gastrointestinal tract). The disintegrant breaks up the tablets and the granules into particles of active and excipients. Superdisintegrants such as croscarmellose sodium, sodium starch glycolate, or crospovidone may also be employed. Of these, sodium starch glycolate is preferred.

The amount of disintegrant is preferably from 1 to 10 %, more preferably from 2 to 8 %, more preferably from 3 to 5 % such as about 4 % w/w.

One or more glidants may be used in the tablet formulation to improve flow. Because of the shape and size of the particles, glidants improve flow in low concentrations. They may be mixed in the final tablet formulation in dry form. Suitable glidants include, for example, alkali metal stearates, colloidal silicon dioxide (including materials sold under the brand names CAB-O-SIL^{®}, SYLOID^{®}, and AEROSIL^{®}), and talc.

The first active ingredient of the formulation is unstable. "Unstable" in this context means that the active ingredient is prone to decompose or react at temperatures above 20 °C and below 30 °C, 40 °C, 50 °C, or 100 °C, and/or that it exhibits significant decomposition after storage at 20 °C for more than 12 months, such as more than 24 months. "Significant decomposition" as used herein means more than 0.05 %, such as more than 0.1 % decomposition.

The first active ingredient is lisinopril. As discussed above, lisinopril is thermally and storage unstable, and known formulations such as those disclosed in EP1765342B1 suffer from the drawbacks of having to use vacuum drying at low temperature to prevent decomposition, and significant decomposition on storage.

Lisinopril is preferably present as a hydrate, most preferably the dihydrate.

Preferably, the first active ingredient is present as a low proportion of the formulation. As used herein, the term "low proportion" means up to 10 %, preferably up to 7 %, more preferably up to 6%, more preferably up to 5% w/w of the total formulation.

The second active ingredient is amlodipine. Amlodipine is preferably present as a salt. Suitable salts include hydrochloride, hydrobromide, sulphate, phosphate or acid phosphate, acetate, maleate, fumarate, lactate, tartrate, citrate, besylate (or besylate ), tosylate and gluconate. Amlodipine besylate is preferred.

Preferably, the second active ingredient is present as a low proportion of the formulation. As used herein, the term "low proportion" means up to 10 %, preferably up to 7 %, more preferably up to 6%, more preferably up to 5% w/w, more preferably up to 3% w/w of the total formulation.

The ratio between the first and second active ingredients may vary according to the desired therapeutic effect. Preferably, the ratio of the first to second active ingredient is between 3:1 and 1:3, such as between 2:1 and 1:2, for example 1:1 on a w/w basis.

The invention encompasses bulk formulations, and unit doses. "Unit dose" refers to physically discrete unit of a therapeutic agent for a subject (e.g., a human patient) to be treated. Each unit contains a predetermined quantity of first and second active ingredients calculated or demonstrated to produce a desired therapeutic effect when administered to a relevant population according to an appropriate dosing regimen. Preferably, the unit dose is a solid unit dose. Preferably, the unit dose is for oral administration.

Solid unit doses include tablets, mini-tablets, capsules, caplets, granules, beads, pellets, and the like. Preferably, the unit dose is a compressed tablet.

Compressed tablets according to the invention may be of any size and weight suitable to be comfortably swallowed by a patient, whilst also delivering the desired dosage of the first and second active ingredients. The tablets may include a coating (e.g. carnauba wax) or be uncoated.

In a preferred embodiment, the first and second active ingredients are not physically separated in the unit dose. As used herein, the term "not physically separated" is intended to mean that the first active ingredient (e.g. lisinopril) and the second active ingredient (e.g. amlodipine) are present together in the same phase of the dosage form.

The weight of the solid unit doses may be between 50 and 1000 mg. Preferably, the weight is between 100 and 500 mg. More preferably, the weight is between 200 and 400 mg, such as 200 mg, 300 mg or 400 mg.

Preferably, the solid unit dose is formulated to comprise between 1 and 50 mg of the first active ingredient per unit dose, such as between 5 and 30 mg, preferably between 10 and 20 mg, such as 10 mg and 20 mg.

Preferably, the solid unit dose is formulated to comprise between 1 and 50 mg of the second active ingredient per unit dose, such as between 2 and 20 mg, preferably between 4 and 15 mg, such as 5 mg and 10 mg.

The invention also relates to methods of treatment of the human or animal body comprising administration of the formulations hereinbefore described. Such methods will depend on the therapeutic nature of the first and second active ingredients.

The formulations of the invention are effective in the treatment of hypertension, preferably systolic or diastolic hypertension. The invention is particularly effective in the treatment of systolic hypertension.

A further aspect of the invention relates to a process for the preparation of a pharmaceutical formulation. As explained above, due to the unexpected stabilising properties of small amounts of dibasic calcium phosphate on unstable active ingredients, less cumbersome processes than those described in the prior art may be deployed.

As related above, the process for the preparation of a pharmaceutical formulation according to the invention comprises the steps of
a) combining the first active ingredient, bulk diluent, and optionally, one or more further adjuvants and/or excipients and homogenizing;
b) adding a binder solution;
c) granulating the product of step b);
d) drying at atmospheric pressure;
e) adding the second active ingredient.

Step a) may be accomplished using any suitable means for mixing. For example, a high or low shear mixer, impeller or chopper. The skilled person will be able to judge when sufficient homogenization has been accomplished. All of the optional further adjuvants and/or excipients may be added at step a), or alternatively, it may be expedient or advantageous to add them at selected subsequent steps.

A binder solution is added at step b). The binder solution is preferably an aqueous solution, and may be water. Preferably, the binder solution comprises a binder as defined above. More preferably, the binder solution comprises partially pregelatinised maize starch, preferably in an amount of from 10 to 40%, such as between 20 and 30 %. The weight of binder solution relative to the homogenized mixture of step a will be typically between 1:10 and 1:1, such as between 1:5 and 1:3.

After addition of the binder solution, the mixture is again mixed until homogeneous. Again, this may be accomplished using any suitable means for mixing, for example, a high or low shear mixer, impeller or chopper.

The mixture from step b) is granulated. This is suitably accomplished in a high shear mixer-granulator, and may occur concurrently with step b).

The granulated mixture is dried under atmospheric conditions. In contrast to the prior art, which teaches that vacuum drying is necessary to avoid decomposition of e.g. lisinopril, the inventors have found this to be unnecessary using the formulations of the present invention. This offers processing advantages, as the use of a vacuum drier is inconvenient and requires additional equipment. Suitably, the granulated mixture is dried on a fluid-bed drier at atmospheric (i.e. ambient) pressure.

Optionally, after the drying step, the mixture can be sieved through a screen to ensure a particular particle size upper limit.

Subsequently, the second active ingredient (amlodipine besylate) is added to the dry granules from step d). A further mixing step may prove necessary or advantageous.

Optionally, after step e), one or more lubricants or tabletting aids may be added to the mixture.

This final blend may be subject to one or more further steps to prepare the desired solid unit dose form. In one preferred embodiment, the blend can be compressed into tablets of appropriate mass on a tableting machine fitted with punches and dies with required shape and size.

After tabletting, the solid unit dose form may optionally be coated with one or more coatings known in the art, such as carnauba wax.

### Examples

### Example 1

Lisinopril/Amlodipine 10 mg/5 mg tablets contain active substances Lisinopril dihydrate and Amlodipine besylate . Lisinopril/Amlodipine 10 mg/5 mg tablets are round, white to off white flat tablets with facet and break mark on one side, debossed with "L A" on the other side.

### Lisinopril/Amlodipine 10 mg/5 mg tablets

| **Active ingredients:** | **Quantity mg/tablet** | **Function** | **Quality reference¹** |
|---|---|---|---|
| Lisinopril | 10.00 | Active substance | Ph.Eur. |
| used as Lisinopril dihydrate | 10.89 | | |
| Amlodipine | 5.00 | Active substance | Ph.Eur. |
| used as Amlodipine besylate | 6.94 | | |

| Other ingredients: | | | |
|---|---|---|---|
| Calcium hydrogen phosphate anhydrous | 5.00 | Stabiliser | Ph.Eur. |
| Mannitol | 146.17 | Filler | Ph.Eur. |
| Partially pregelatinised maize starch | 20.00 | Binder | Ph.Eur. |
| Sodium starch glycolate type A | 8.00 | Disintegrant | Ph.Eur. |
| Magnesium stearate | 3.00 | Lubricant | Ph.Eur. |
| Water purified² | / | Granulating aid | Ph.Eur. |
| Total weight | 200.00 mg | | |

| | | | |
|---|---|---|---|
| ¹ references: current edition of the Pharmacopoeia. ² Water, purified is used as granulating aid, but is not included in the final composition of the product. Calcium hydrogen phosphate anhydrous - DI-CAFOS A 12 (Budenheim, Vienna, Austria) having particle size less than 45 µM. | | | |

### Lisinopril/Amlodipine 20 mg/10 mg tablets

| **Active ingredients:** | **Quantity mg/tablet** | **Function** | **Quality reference¹** |
|---|---|---|---|
| Lisinopril | 20.00 | Active substance | Ph.Eur. |
| used as Lisinopril dihydrate | 21.78 | | |
| Amlodipine | 10.00 | Active substance | Ph.Eur. |
| used as Amlodipine besylate | 13.88 | | |

| **Other ingredients:** | | | |
|---|---|---|---|
| Calcium hydrogen phosphate anhydrous | 10.00 | Stabiliser | Ph.Eur. |
| Mannitol | 292.34 | Filler | Ph.Eur. |
| Partially pregelatinised maize starch | 40.00 | Binder | Ph.Eur. |
| Sodium starch glycolate type A | 16.00 | Disintegrant | Ph.Eur. |
| Magnesium stearate | 6.00 | Lubricant | Ph.Eur. |
| Water purified² | / | Granulating aid | Ph.Eur. |
| **Total weight** | **400.00 mg** | | |

| | | | |
|---|---|---|---|
| Calcium hydrogen phosphate anhydrous - DI-CAFOS A 12 (Budenheim, Vienna, Austria) having particle size less than 45 µM. | | | |

The manufacturing process comprises steps of mixing, wet granulation and lubrication of common blend granulate of Lisinopril/Amlodipine, dividing the blend into two portions and tablet compression of 20 mg/10 mg and 10 mg/5 mg tablets or compression of the blend in one tablet strength.

### STEP I - WEIGHING

All ingredients were weighed separately and checked qualitatively and quantitatively against the manufacturing formula.

### STEP II - MIXING (PRE-BLEND)

Active ingredient and excipients were mixed in a high-shear mixer granulator until homogenisation:

| | |
|---|---|
| Lisinopril dihydrate | 4.356 kg |
| Calcium hydrogen phosphate anhydrous | 2.000 kg |
| Mannitol | 58.468 kg |
| Partially pregelatinized maize starch | 5.600 kg |
| Sodium starch glycolate type A | 3.200 kg |

### STEP III WET GRANULATION AND SCREENING

The following quantities of water and excipient are put in the stainless steel vessel for preparation of the binder solution:

| | |
|---|---|
| Water purified | 17.500 kg |
| Partially pregelatinised maize starch | 2.400 kg |

The binder solution was mixed until complete homogenization was acheived. The mixture from step II was granulated in a high shear mixer-granulator with the addition of the binder solution and wet mass was screened through selected wet screening mill.

### STEP IV - DRYING (PC 1)

The screened wet granulate was dried in a fluid-bed dryer.

### STEP V - SCREENING (PC 2)

Dried granulate was passed through a suitable screen with a pre-determined pore size.

### STEP VI - MIXING (Blend)

The active ingredient Amlodipine besylate was mixed with previously obtained granulate from phase V.

| | |
|---|---|
| Amlodipine besylate | 2.776 kg |

### STEP VII -MIXING (Final blend) (PC 3)

The blend with Amlodipine besylate from step VI was lubricated with the previously sieved:

| | |
|---|---|
| Magnesium stearate | 1.200 kg |

The holding time of the final blend was defined as a period of 15 days. The respective holding period of the bulk product was supported by appropriate stability indicating hold time report, performed on two different product batches.

### STEP VIII - TABLET COMPRESSION (PC 4)

The final blend was divided in two portions, one for each tablet strength: 20 mg/10 mg and 10 mg/5 mg tablets, or can be fully compressed into tablets of one strength on a tableting machine fitted with punches and dies with required shape and size according to the division plan:

| | | |
|---|---|---|
| Lisinopril/Amlodipine 20 mg/10 mg tablets | min | 55.000 kg blend or 137 500 tablets |
| | max | 80.000 kg blend or 200 000 tablets |
| Lisinopril/Amlodipine 10 mg/5 mg tablets | min | 25.000 kg blend or 125 000 tablets |
| | max | 80.000 kg blend or 400 000 tablets |

Upon manufacturing, bulk tablets were transferred and sealed into polyethylene bags and immediately placed together with silica into stainless steel drums. The bulk tablets were stored in the warehouse until packaging under monitored temperature and humidity conditions.

The holding time of Lisinopril/Amlodipine tablets is defined in a period of 6 months.

### Example 2

### Lisinopril/Amlodipine 20 mg/5 mg tablets

| **Active ingredients:** | **Quantity mg/tablet** | **Function** | **Quality reference¹** |
|---|---|---|---|
| Lisinopril | 20.00 | Active substance | Ph.Eur. |
| used as Lisinopril dihydrate | 21.78 | | |
| Amlodipine | 5.00 | Active substance | Ph.Eur. |
| used as Amlodipine besylate | 6.94 | | |

| **Other ingredients:** | | | |
|---|---|---|---|
| Calcium hydrogen phosphate anhydrous | 10.00 | Stabiliser | Ph.Eur. |
| Mannitol | 299.28 | Filler | Ph.Eur. |
| Partially pregelatinised maize starch | 40.00 | Binder | Ph.Eur. |
| Sodium starch glycolate type A | 16.00 | Disintegrant | Ph.Eur. |
| Magnesium stearate | 6.00 | Lubricant | Ph.Eur. |
| Water purified² | / | Granulating aid | Ph.Eur. |
| **Total weight** | **400.00 mg** | | |

| | | | |
|---|---|---|---|
| Calcium hydrogen phosphate anhydrous - DI-CAFOS A 12 (Budenheim, Vienna, Austria) having particle size less than 45 µM. ¹ references: current edition of the Pharmacopoeia. ² Water, purified is used as granulating aid, but it is not included in the final composition of the product. | | | |

The manufacturing process is composed of mixing, wet granulation lubrication and tablet compression of Lisinopril/Amlodipine 20 mg/5 mg tablets.

### STEP I - WEIGHING

All ingredients were weighed separately and checked qualitatively and quantitatively against the manufacturing formula.

### STEP II - MIXING (PRE-BLEND)

Active ingredient and excipients were mixed in a high-shear mixer granulator until homogenized.

| | |
|---|---|
| Lisinopril dihydrate | 4.356 kg |
| Calcium hydrogen phosphate anhydrous | 2.000 kg |
| Mannitol | 59.856 kg |
| Partially pregelatinized maize starch | 5.600 kg |
| Sodium starch glycolate type A | 3.200 kg |

### STEP III WET GRANULATION AND SCREENING

Following quantity of water and excipient were put in the stainless vessel for preparation of the binder solution:

| | |
|---|---|
| Water purified | 17.820 kg |
| Partially pregelatinised maize starch | 2.400 kg |

The binder solution was mixed until its complete homogenization was acheived. The mixture from step II was granulated in a high shear mixer-granulator with the addition of the binder solution and wet mass is screened through selected wet screening mill.

### STEP IV - DRYING

The screened wet granulate was dried in a fluid-bed dryer.

### STEP V - SCREENING (PC 2)

Dried granulate was passed through a suitable screen with pre-determined pore size.

### STEP VI - MIXING (Blend)

The active ingredient Amlodipine besylate was mixed with previously obtained granulate from phase V.

| | |
|---|---|
| Amlodipine besylate | 1.388 kg |

### STEP VII -MIXING (Final blend)

The blend with Amlodipine besylate from the phase VI was lubricated with the previously sieved:

| | |
|---|---|
| Magnesium stearate | 1.200 kg |

### STEP VIII - TABLET COMPRESSION

The final blend was compressed into tablets on a rotary tablet press machine fitted with punches and dies with required shape and size.

Upon manufacturing, bulk tablets are transferred and sealed into polyethylene bags and immediately placed together with silica into stainless steel drums. The bulk tablets are stored in the warehouse until packaging under monitored temperature and humidity conditions.

The holding time of Lisinopril/Amlodipine 20 mg/5 mg tablets was defined as a period of 6 months.

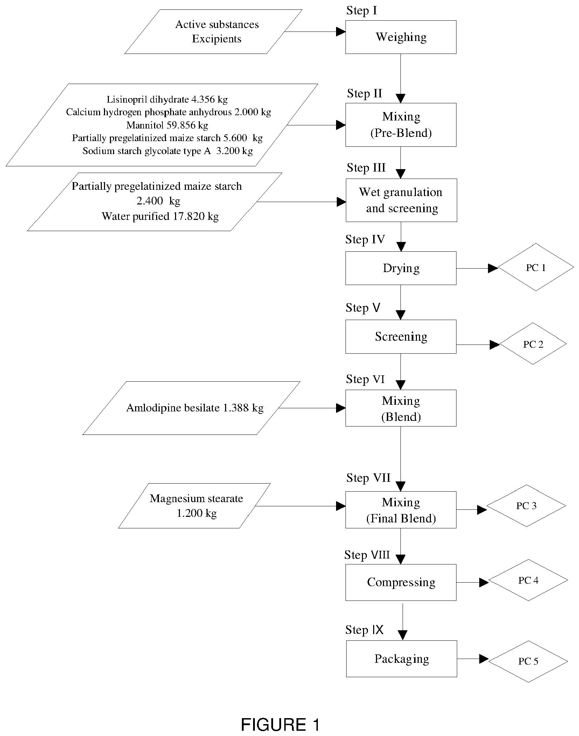

Figure 1 is a flow chart for production of standard batch size of 80.000 kg granulate or 200 000 tablets of Example 2.

### Example 3

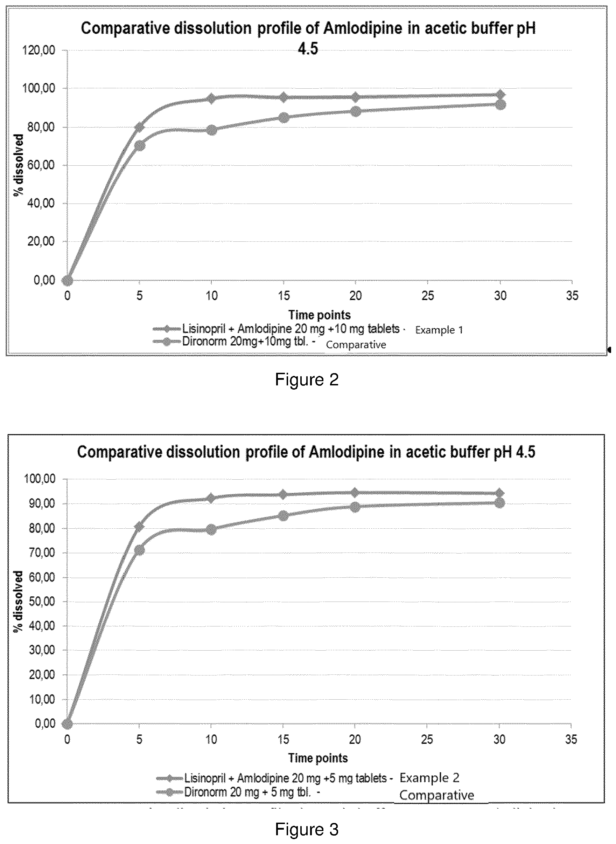

A comparative dissolution study of Lisinopril/Amlodipine 20 mg/10 mg tablets according to the invention (Example 1) versus Dironorm 20 mg/10 mg tablets according to EP1765342B1 was conducted in acetic buffer pH 4.5. Results are shown in Figure 2.

A comparative dissolution study of Lisinopril/Amlodipine 20 mg/5 mg tablets according to the invention (Example 2) versus Dironorm 20 mg/5 mg tablets according to EP1765342B1 was conducted in acetic buffer pH 4.5. Results are shown in Figure 3.

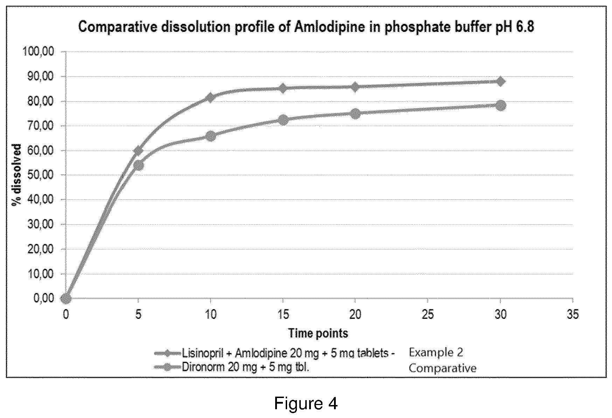

A comparative dissolution study of Lisinopril/Amlodipine 20 mg/10 mg tablets according to the invention (Example 1) versus Dironorm 20 mg/10 mg tablets according to EP1765342B1 was conducted in phosphate buffer pH 6.8. Results are shown in Figure 4.

The results show that tablets prepared according to Example 1 and Example 2 of the invention show faster and more complete dissolution of amlodipine at different pH values compared to the prior art formulations.

### Example 4

### Impurity profile comparison

The impurity profiles of Lisinopril/Amlodipine tablets according to Examples 1 and 2 of the invention and comparative Lisinopril/Amlodipine tablets according to EP1765342B1 were analysed after two year's storage:

**Table 1 Lisinopril impurity profiles of Lisinopril/Amlodipine 10 mg/5 mg tablets according to invention and prior art**

| | **Lisinopril/Amlodipine 10 mg/5 mg tablets** | **Lisinopril/Amlodipine 10 mg/5 mg tablets** |
|---|---|---|
| | EXAMPLE 1 | EP1765342B1 |
| - Impurity C (S,S,S-DKP): | 0.05% | 0.15% |
| - Any other unspecified impurity: | 0.06% | 0.05% |
| | 0.2% | 0.3% |
| - Total impurities: | | |

| | | |
|---|---|---|
| *Results from stability study, Long-term storage conditions: 25°C/60% RH | | |

**Table 2 Lisinopril impurity profiles of Lisinopril/Amlodipine 20 mg/10 mg tablets and Dironorm 20 mg/10 mg tablets**

| | **Lisinopril/Amlodipine 20 mg/10 mg tablets** | **Lisinopril/Amlodipine 20 mg/10 mg tablets** |
|---|---|---|
| | EXAMPLE 1 | EP1765342B1 |
| - Impurity C (S,S,S-DKP): | 0.06% | 0.15% |
| - Any other unspecified impurity: | 0.06% | 0.10% |
| | 0.2% | 0.3% |
| - Total impurities: | | |

| | | |
|---|---|---|
| *Results from stability study, Long-term storage conditions: 25°C/60% RH | | |

**Table 3 Lisinopril impurity profiles of Lisinopril/Amlodipine 20 mg/5 mg tablets according to invention and prior art**

| | **Lisinopril/Amlodipine 20 mg/5 mg tablets** | **Lisinopril/Amlodipine 20 mg/5 mg tablets** |
|---|---|---|
| | EXAMPLE 2 | EP1765342B1 |
| - Impurity C (S,S,S-DKP): | BDL | 0.13% |
| - Any other unspecified impurity: | 0.07% | BDL |
| | 0.1% | 0.2% |
| - Total impurities: | | |

| | | |
|---|---|---|
| BDL = below disregard limit (0.05*%*) *Results from stability study, Long-term storage conditions: 25°C/60% RH | | |

These results show that the tablets prepared according to the invention show a lower total impurity level than the equivalent tablets of the prior art. In particular, the level of lisinopril S,S,S-diketopiperazine (S,S,S-DKP) is significantly lower in the inventive compositions than in those known from EP1765342B1.

### Example 5

The solubility of the inventive tablets according to Examples 1 and 2 was compared to the formulations taught in Int. J. Pharm. Sci. Rev. Res., 20(1) ("Peikova*").*

The inventive tablets were found to have better solubility in comparison with the products in *Peikova* under the same conditions (500 ml medium with pH = 1.2, 50 rpm). The solubility of both active ingredients in the formulations of *Peikova* is between 60 - 70 % within 15 min; in comparison the inventive products of Examples 1 and 2, showed a solubility of both active ingredients of more than 85% within 15 min.

This is advantageous, especially for Lisinopril who has lower permeability (BCS class III) and resultant higher inter-individual variability in the pharmacokinetic parameters among patients.

### Example 6

### Effect of dicalcium phosphate anhydrous in Lisinopril/Amlodipine tablets

In order to evaluate the stability of formulation with and without dicalcium phosphate anhydrous, Lisinopril/Amlodipine tablets 20 mg/10 mg were prepared and positioned in stability chambers at 25°C/60% relative humidity for 24 months.

**TABLE 4: Composition of trial formulations**

| | Lisinopril/ Amlodipine 20 mg/10 mg tablets | |
|---|---|---|
| | Comparative example A | Example 1 |
| **Ingredient** | Quantity (% w/w) | |
| Lisinopril (as dihydrate) | 5.45 | 5.45 |
| Dicalcium phosphate anhydrous | / | 2.50 |
| Mannitol | 72.58 | 73.58 |
| Partially pregelatinised maize starch | 10.00 | 10.00 |
| Sodium starch glycolate type A | 7.00 | 4.00 |
| Amlodipine (as besylate ) | 3.47 | 3.47 |
| Magnesium stearate | 1.50 | 1.50 |

| | | |
|---|---|---|
| Calcium hydrogen phosphate anhydrous - DI-CAFOS A 12 (Budenheim, Vienna, Austria) having particle size less than 45 µM. | | |

The products were stored at 25°C/60% relative humidity for 30 days, and the impurity profile analysed by HPLC. Degradation products deriving from Amlodipine were detected as follows:

**TABLE 5: Degradation products deriving from Amlodipine**

| | **Comparative example A** | **Example 1** |
|---|---|---|
| **Impurity** | **%** | **%** |
| RRT 0.16 | 0.01 | ND |
| RRT 0.47 | 0.02 | ND |
| Impurity B | ND | 0.002 |
| Impurity D | 0.01 | 0.01 |
| Impurity E | 0.01 | ND |
| Impurity F | 0.03 | 0.02 |
| RRT 1.30 | 0.004 | ND |
| RRT 1.40 | 0.004 | ND |
| RRT 1.48 | 0.01 | ND |
| RRT 1.65 | 0.01 | ND |
| Deschloro impurity | 0.01 | 0.01 |
| **TOTAL** | **0.10** | **0.04** |

| | | |
|---|---|---|
| RRT = relative retention time; ND = not detected | | |

Degradation products deriving from lisinopril were detected as shown in Table 6.

**TABLE 6: Degradation products deriving from Lisinopril**

| | **Comparative example A** | **Example 1** |
|---|---|---|
| **Impurity** | **%** | **%** |
| RRT 0.63 | 0.04 | ND |
| Impurity A | 0.03 | 0.01 |
| Impurity E | 0.14 | 0.03 |
| Impurity C | 0.07 | 0.06 |
| RRT 3.05 | 0.06 | 0.05 |
| **TOTAL** | **0.33** | **0.16** |

Conclusion - the formulation of the invention, containing 2.5 % w/w dicalcium phosphate anhydrous, displayed markedly less decomposition of both lisinopril and amlodipine than the comparative formulation A lacking dicalcium phosphate anhydrous.

## Claims

1. A pharmaceutical formulation comprising
a) a first active ingredient which is lisinopril or a salt or hydrate thereof;
b) a second active ingredient which is amlodipine or a salt or hydrate thereof;
c) a bulk diluent;
d) optionally, one or more further adjuvants and/or excipients; and
e) dibasic calcium phosphate anhydrous in an amount of from 2 to 5 % w/w.

2. A pharmaceutical formulation according to claim 1 wherein dibasic calcium phosphate anhydrous is present in an amount of from 2 % to 3 % w/w.

3. A pharmaceutical formulation according to claim 1 or 2 wherein the median (D₅₀) particle size of the dibasic calcium phosphate anhydrous is from 1 to 10 µm, wherein the median particle size is measured by a laser diffraction/scattering particle size distribution analyzer.

4. A pharmaceutical formulation according to any preceding claim wherein the bulk diluent is a polyol, preferably wherein the polyol is selected from the group of isomalt, lactitol, mannitol, sorbitol and xylitol, more preferably wherein the polyol is mannitol.

5. A pharmaceutical formulation according to any preceding claim further comprising one or more binders, disintegrants, lubricants, fillers or granulating aids.

6. A pharmaceutical formulation according to any preceding claim wherein the first and second active ingredients are present in a ratio of between 1:3 and 3:1.

7. A pharmaceutical formulation according to any preceding claim in the form of a compressed tablet, preferably wherein the tablet has a mass of between 50 and 500 mg.

8. A pharmaceutical formulation according to claim 7 comprising the first active ingredient in an amount of between 5 and 25 mg per tablet.

9. A pharmaceutical formulation according to any one of claims 7 to 8 comprising the second active ingredient in an amount of between 5 and 25 mg per tablet.

10. A pharmaceutical formulation according to any preceding claim for use in medicine.

11. A pharmaceutical formulation according to any one of claims 1 to 9 for the treatment of hypertension.

12. A process for the preparation of a pharmaceutical formulation as claimed in any one of claims 1 to 9, comprising the steps of
a) combining the first active ingredient, bulk diluent, and optionally, one or more further adjuvants and/or excipients and homogenizing;
b) adding a binder solution;
c) granulating the product of step b);
d) drying at atmospheric pressure;
e) adding the second active ingredient to form a bulk formulation.

13. The process according to claim 12 comprising a further step of adding a lubricant.

14. The process of claim 12 or 13 comprising a further step of forming the bulk formulation into tablets.

15. The process of claim 14 comprising the further step of coating the tablets.

## Patentansprüche

1. Pharmazeutische Formulierung, die Folgendes umfasst:
a) einen ersten Wirkstoff, der Lisinopril oder ein Salz oder Hydrat davon ist;
b) einen zweiten Wirkstoff, der Amlodipin oder ein Salz oder Hydrat davon ist;
c) ein füllendes Verdünnungsmittel;
d) optional ein oder mehrere weitere Adjuvanzien und/oder Hilfsstoffe; und
e) wasserfreies zweibasisches Calciumphosphat in einer Menge von 2 bis 5 m/m-%.

2. Pharmazeutische Formulierung nach Anspruch 1, wobei wasserfreies zweibasisches Calciumphosphat in einer Menge von 2 m/m-% bis 3 m/m-% vorliegt.

3. Pharmazeutische Formulierung nach Anspruch 1 oder 2, wobei die mediane (D₅₀) Partikelgröße des wasserfreien zweibasischen Calciumphosphats von 1 bis 10 µm beträgt, wobei die mediane Partikelgröße durch ein Laserbeugungs-/Laserstreuungs-Partikelgrößenverteilungs-Analysegerät gemessen wird.

4. Pharmazeutische Formulierung nach einem vorstehenden Anspruch, wobei das füllende Verdünnungsmittel ein Polyol ist, bevorzugt wobei das Polyol aus der Gruppe von Isomalt, Lactitol, Mannitol, Sorbitol und Xylitol ausgewählt ist, bevorzugter wobei das Polyol Mannitol ist.

5. Pharmazeutische Formulierung nach einem vorstehenden Anspruch, die weiter ein oder mehrere Bindemittel, Sprengmittel, Schmiermittel, Füllstoffe oder Granulierhilfsmittel umfasst.

6. Pharmazeutische Formulierung nach einem vorstehenden Anspruch, wobei der erste und zweite Wirkstoff in einem Verhältnis von zwischen 1 : 3 und 3 : 1 vorliegen.

7. Pharmazeutische Formulierung nach einem vorstehenden Anspruch in der Form einer gepressten Tablette, bevorzugt wobei die Tablette eine Masse von zwischen 50 und 500 mg aufweist.

8. Pharmazeutische Formulierung nach Anspruch 7, die den ersten Wirkstoff in einer Menge von zwischen 5 und 25 mg pro Tablette umfasst.

9. Pharmazeutische Formulierung nach einem der Ansprüche 7 bis 8, die den zweiten Wirkstoff in einer Menge von zwischen 5 und 25 mg pro Tablette umfasst.

10. Pharmazeutische Formulierung nach einem vorstehenden Anspruch für die Verwendung in der Medizin.

11. Pharmazeutische Formulierung nach einem der Ansprüche 1 bis 9 für die Behandlung von Bluthochdruck.

12. Verfahren für die Herstellung einer pharmazeutischen Formulierung nach einem der Ansprüche 1 bis 9, das die folgenden Schritte umfasst:
a) Vereinigen des ersten Wirkstoffs, füllenden Verdünnungsmittels und optional eines oder mehrerer weiterer Adjuvanzien und/oder Hilfsstoffe und Homogenisieren;
b) Zugeben einer Bindemittellösung;
c) Granulieren des Produkts von Schritt b);
d) Trocknen bei Atmosphärendruck;
e) Zugeben des zweiten Wirkstoffs, um eine Formulierungsmasse zu bilden.

13. Verfahren nach Anspruch 12, das einen weiteren Schritt des Zugebens eines Schmiermittels umfasst.

14. Verfahren nach Anspruch 12 oder 13, das einen weiteren Schritt des Formens der Formulierungsmasse zu Tabletten umfasst.

15. Verfahren nach Anspruch 14, das den weiteren Schritt des Beschichtens der Tabletten umfasst.

## Revendications

1. Formulation pharmaceutique, comprenant
a) un premier ingrédient actif qui est le lisinopril ou un sel ou hydrate de celui-ci ;
b) un deuxième ingrédient actif qui est l'amlodipine ou un sel ou hydrate de celle-ci ;
c) un diluant en vrac ;
d) éventuellement, un ou plusieurs autres adjuvants et/ou excipients ; et
e) du phosphate de calcium dibasique anhydre selon une quantité allant de 2 à 5% p/p.

2. Formulation pharmaceutique selon la revendication 1, dans laquelle le phosphate de calcium dibasique anhydre est présent selon une quantité allant de 2% à 3% p/p.

3. Formulation pharmaceutique selon la revendication 1 ou 2, dans laquelle la taille des particules moyenne (D₅₀) du phosphate de calcium dibasique anhydre va de 1 à 10 µm, où la taille des particules moyenne est mesurée à l'aide d'un analyseur de distribution granulométrique par diffraction/diffusion laser.

4. Formulation pharmaceutique selon l'une quelconque des revendications précédentes, dans laquelle le diluant en vrac est un polyol, préférablement où le polyol est choisi dans le groupe constitué par l'isomalt, le lactitol, le mannitol, le sorbitol et le xylitol, plus préférablement où le polyol est le mannitol.

5. Formulation pharmaceutique selon l'une quelconque des revendications précédentes, comprenant en outre un(e) ou plusieurs liants, délitants, lubrifiants, charges ou auxiliaires de granulation.

6. Formulation pharmaceutique selon l'une quelconque des revendications précédentes, dans laquelle le premier et le deuxième ingrédient actif sont présents selon un rapport compris entre 1:3 et 3:1.

7. Formulation pharmaceutique selon l'une quelconque des revendications précédentes, sous la forme d'un comprimé, préférablement où le comprimé présente une masse comprise entre 50 et 500 mg.

8. Formulation pharmaceutique selon la revendication 7, comprenant le premier ingrédient actif selon une quantité comprise entre 5 et 25 mg par comprimé.

9. Formulation pharmaceutique selon l'une quelconque des revendications 7 à 8, comprenant le deuxième ingrédient actif selon une quantité comprise entre 5 et 25 mg par comprimé.

10. Formulation pharmaceutique selon l'une quelconque des revendications précédentes, pour une utilisation en médecine.

11. Formulation pharmaceutique selon l'une quelconque des revendications 1 à 9, pour le traitement de l'hypertension.

12. Procédé de préparation d'une formulation pharmaceutique selon l'une quelconque des revendications 1 à 9, comprenant les étapes
a) d'association du premier ingrédient actif, du diluant en vrac, et éventuellement, d'un ou plusieurs autres adjuvants et/ou excipients, et d'homogénéisation ;
b) d'addition d'une solution de liant ;
c) de granulation du produit de l'étape b) ;
d) de séchage à pression atmosphérique ;
e) d'addition du deuxième ingrédient actif afin de former une formulation en vrac.

13. Procédé selon la revendication 12, comprenant une étape supplémentaire d'addition d'un lubrifiant.

14. Procédé selon la revendication 12 ou 13, comprenant une étape supplémentaire de mise en forme de la formulation en vrac en comprimés.

15. Procédé selon la revendication 14, comprenant l'étape supplémentaire d'enrobage des comprimés.
